# EUROPÄISCHE PATENTSCHRIFT

(11) **EP 1 007 741 B1**
(45) Veröffentlichungstag und Bekanntmachung des Hinweises auf die Patenterteilung: **24.04.2002**
(21) Anmeldenummer: 98954114.9
(22) Anmeldetag: 04.09.1998
(51) Int. Cl.: C12Q 1/68, G09F 3/00

(54) **VERFAHREN ZUR MARKIERUNG VON FESTEN, FLÜSSIGEN ODER GASFÖRMIGEN SUBSTANZEN**
METHOD FOR MARKING SOLID, LIQUID OR GASEOUS SUBSTANCES
PROCEDE DE MARQUAGE DE SUBSTANCES SOLIDES, LIQUIDES OU GAZEUSES

(30) Priorität: 05.09.1997 DE 19738816
(43) Veröffentlichungstag der Anmeldung: 14.06.2000
(73) Patentinhaber: november Aktiengesellschaft Gesellschaft für Molekulare Medizin, 91056 Erlangen (DE)
(72) Erfinder: BERTLING, Wolf, D-91056 Erlangen (DE); KOSAK, Hans, D-53123 Bonn (DE)
(74) Vertreter: Gassner, Wolfgang, Dr.
(86) Internationale Anmeldenummer: DE9802615
(87) Internationale Veröffentlichungsnummer: WO9913102

(56) Entgegenhaltungen:
- WO-A-87/06383
- WO-A-90/14441
- WO-A-91/17265
- WO-A-94/04918
- WO-A-95/02702
- WO-A-96/17954
- WO-A-96/19586
- WO-A-97/32040
- WO-A-98/55657
- US-A- 5 139 812
- SANO T ET AL: "Deoxyribonucleic acids as unique markers in molecular detection" GENETIC ANALYSIS: BIOMOLECULAR ENGINEERING, Bd. 14, Nr. 2, Juli 1997, Seite 37-40 XP004126263

## Beschreibung

Die Erfindung betrifft ein Verfahren nach dem Oberbegriff des Anspruchs 1. Sie betrifft ferner einen Kit zur Durchführung des Verfahrens nach Anspruch 1.

Ein solches Verfahren ist aus der US 5,643,728 bekannt. Dabei ist die Markierung in einem bestimmten Sequenzabschnitt einer Nukleinsäuresequenz enthalten. Zur Identifikation der Markierung wird die Nukleinsäuresequenz unter Anwendung der Polymerase-Ketten-Raektion (PCR) vervielfältigt. Anschließend wird die im vervielfältigten Produkt enthaltende Markierung mittels Sequenzierung identifiziert.

Ein weiteres Verfahren ist aus der DE 44 39 896 A1 bekannt. Dabei beherbergen die der zu markierenden Substanz zugefügten DNA-Moleküle einen Längenpolymorphismus, welcher die Markierung trägt. Zur Identifikation der Markierung werden die DNA-Moleküle mittels PCR vervielfältigt und dann einer Gel-Elektrophorese unterzogen. Die als Ergebnis der Gel-Elektopohrese beobachtbare Bandensequenz repräsentiert ähnlich einem Strichcode die Markierung. Die Bandensequenz kann auch in eine Zahl übersetzt werden.

Die vorerwähnten Verfahren sind in mehrfacher Hinsicht nachteilig:
a) Die Verfahren sind zeit- und kostenaufwendig, weil zur Identifikation der Markierung sowohl eine PCR als auch eine Gel-Elektroporese durchgeführt werden müssen;
b) die Verfahren sind schwer automatisierbar, weil die bei der PCR gebildeten Reaktionsprodukte zur Durchführung der Gel-Elektrophorese auf ein Gel übertragen werden müssen;
c) die Verfahren sind fehleranfällig, weil zu deren Durchführung eine Vielzahl von kontaminationsempfindlichen Pipettiervorgängen erforderlich sind;
d) die Verfahren sind aufwendig, weil zu einer Markierung einer Vielzahl von Substanzen die gleiche Anzahl an Markierungs-DNAs hergestellt werden muß.

Ein weiteres Verfahren ist aus der US 5,139,812 bekannt. Dabei wird eine vorgegebene Nukleinsäuresequenz enthaltende Tinte zur fälschungssicheren Markierung von Gegenständen verwendet. Um eine Mehrzahl von Gegenständen unterscheidbar zu markieren, werden mit der Tinte unterschiedliche Beschriftungen aufgebracht. Zur Identifikation einer solchermaßen aufgebrachten Markierung wird die Beschriftung mittels einer Farbreaktion sichtbar gemacht. Die Identifikation kann auch durch eine radioaktive Markierung der verwendeten Nukleinsäuresequenz erfolgen. - Dieses Verfahren ist vor allem deshalb nachteilig, weil die Aufbringung einer unterscheidbaren Markierung umständlich ist und zur Identifikation der markierte Gegenstand zerstört werden muß.

Aus der WO 95/17737 ist es weiterhin bekannt, zur Markierung von Kunstgegenständen das Blut des Künstlers zu verwenden. Zur Identifikation wird die Markierung mit einer hinterlegten weiteren Blutprobe mittels DNA-Analyse verglichen. - Dieses Verfahren ermöglicht nicht ohne weiteres die Herstellung einer Vielzahl unterschiedlicher Markierungen. Die Indentifikation ist aufwendig.

Aufgabe der Erfindung ist es, ein Verfahren und einen Kit zu dessen Durchführung anzugeben, mit dem eine Vielzahl von Substanzen oder Gegenständen fälschungssicher und unterscheidbar markier- und nachfolgend kostengünstig und schnell identifizierbar ist.

Diese Aufgabe wird durch die Merkmale der Ansprüche 1 und 20 gelöst. Zweckmäßige Ausgestaltungen der Erfindung ergeben sich aus den Merkmalen der Ansprüche 2 bis 19 und 21 bis 27.

Nach der verfahrensseitigen Lösung der Erfindung ist vorgesehen, daß zur Identifikation eine erste Primergruppe mit einem zum ersten Sequenzabschnitt korrespondierenden ersten Primerabschnitt und eine zweite Primergruppe mit einem zum dritten Sequenzabschnitt korrespondierenden dritten Primerabschnitt verwendet wird, wobei jede der Primergruppen vier, sich jeweils in mindestens einer zusätzlichen endständig vorgesehen Base unterscheidende Primervarianten umfaßt, so daß genau eine Primervariante der ersten Primergruppe zusammen mit genau einer Primervariante der zweiten Primergruppe eine Amplifikation der Nukleinsäuresequenz ermöglicht.

Mit dem erfindungsgemäßen Verfahren kann auf einfache Weise eine unterscheidbare Markierung bzw, innere Numerierung einer Vielzahl von Gegenständen erfolgen. Es können mit einer relative kleinen Anzahl unterschiedlicher Markierungs- bzw. Nukleinsäuresequenzen eine große Anzahl an unterschiedlichen Markierungen bereitgestellt werden. Die Markierungen sind schnell und ohne großen Aufwand identifizierbar.

Nach einer Ausgestaltung der Erfindung weist die Nukleinsäuresequenz mehr als 20, vorzugsweise 40 Nukleotide, auf.

Zur Markierung werden vorteilhafterweise Nukleinsäuresequenzen verwendet, die im zweiten Sequenzabschnitt sich unterscheiden. Dieser besteht vorteilhafterweise aus zwei Teilbereichen, von denen jeder mit mindestens einer Base besetzt ist. Die Kombination der beiden Teilbereiche ermöglicht die Darstellung von insgesamt sechzehn verschiedenen Nukleinsäurevarianten, nämlich Adenin (=A) - A, A - Thymidin (=T), A - Guanin (=G), A - Cytosin (=C), T - A, T - T, T - G, T - C, G - A, G - T, G - G, G - C, C - A, C - T, C - G, C - C. Die 16 Nukleinsäurevarianten bilden einen Nukleinsäuresatz.

Vorteilhafterweise ist der zweite Sequenzabschnitt aus zwei Teilbereichen gebildet, von denen mindestens einer aus einer Mehrzahl Basen besteht. Dementsprechend ist dann die korrespondierende Primervariante endständig mit derselben Anzahl korrespondierender Basen versehen. Dadurch wird die Wahrscheinlichkeit der Bildung von falschpositiven Proben drastisch reduziert. Der Teilbereich kann aus einer spezifischen Sequenz oder aus mehreren identischen Basen bestehen.

Zur Erhöhung der Kombinationsmöglichkeiten können weitere Nukleinsäuresätze verwendet werden, deren Nukleinsäurevarianten sich im ersten und dritten Sequenzabschnitt unterscheiden. Z.B. können zur Erhöhung der Kombinationsmöglichkeiten von 16 auf 16² die vorbeschriebenen Nukleinsäurevarianten eines Nukleinsäuresatzes mit weiteren Nukleinsäurevarianten kombiniert werden, die aus einem zweiten Nukleinsäuresatz ausgewählt sind. Der zweite Nukleinsäuresatz unterscheidet sich vom ersten Nukleinsäuresatz im ersten und dritten Sequenzabschnitt. Analog kann die Kodierungskapazität durch die Verwendung von n Nukleinsäuresätzen auf 16ⁿ erhöht werden.

Der erste und der dritte Sequenzabschnitt weisen vorzugsweise die gleiche Anzahl von Nukleotiden auf. Die erste und der dritte Sequenzabschnitt sind zweckmäßigerweise so ausgebildet, daß sie unter vergleichbaren Stringenzbedingungen aufschmelzbar. - Das ermöglicht eine sehr einfache Amplifikation mittels Polymerase- (PCR) oder Ligase-Ketten-Reaktion (LCR).

Als Nukleinsäuresequenzen können selbstverständlich auch Nukleinsäurederivatsequenzen, insbesondere proteinartige Nukleinsäure (PNA) oder Phosphortionatnukleinsäuren (PTO) oder Hybride davon, verwendet werden. Derartige Nukleinsäurederivatsequenzen zeichnen sich zum Teil durch eine verbesserte Stabilität aus.

Zum Schutz einer oder mehrerer auf feste Gegenstände aufgetragenen Nukleinsäuresequenz/en kann/können diese durch eine Schutzschicht, wie Wachs, abgedeckt sein. Die Nukleinsäuresequenz/en kann/können weiterhin Bestandteil einer auf feste Gegenstände aufgetragenen Schicht, wie einem Lack, sein. Auch durch Impregnation oder Mischung kann eine Markierung bewirkt werden.

Zur Identifikation der mindestens einen Nukleinsäuresequenz wird diese zweckmäßigerweise extrahiert und eine die Nukleinsäuresequenz enthaltende Lösung hergestellt. Die Nukleinsäuresequenz kann dann unter Verwendung der Primervarianten mittels PCR oder LCR vervielfältigt werden. Die vervielfältigte Nukleinsäuresequenz bzw. das Amplifikat wird zweckmäßigerweise nachfolgend mittels Fluoreszenz, DNA, Gel-Elektrophorese, Restriktionsanalyse, Hybridisierung oder mittels Sequenzierung nachgewiesen. Der Nachweis mittels Fluoreszenz ist besonders einfach und schnell durchzuführen. Er kann direkt in einer Mikrotiterplatte erfolgen.

Erfindungsgemäß ist zur Durchführung des Verfahrens ein Kit zur Identifikation einer Markierung, wobei der Kit eine erste Primergruppe mit einem zum ersten Sequenzabschnitt korrespondierenden ersten Primerabschnitt und eine zweite Primergruppe mit einem zum dritten Sequenzabschnitt korrespondierenden dritten Primerabschnitt aufweist, wobei jede der Primergruppen vier sich jeweils in mindestens einer zusätzlichen endständig vorgesehen Base unterscheidende Primervarianten umfaßt, so daß genau eine Primervariante der ersten Primergruppe zusammen mit genau einer Primervariante der zweiten Primergruppe zur Nukleinsäuresequenz komplementär ist.

Vorteilhafterweise weisen die Primervarianten dieselbe oder eine ähnliche Anzahl an Nukleotiden auf. Sie können insbesondere mehr als 10 Nukleotide, vorzugsweise 20 Nukleotide, aufweisen. Insbesondere sind alle Primervarianten mit der Nukleinsäuresequenz unter ähnlichen oder vergleichbaren Stringenzbedingungen aufschmelzbar.

Zur Erleichterung des Handlings kann jede der Primervarianten separat von einem Trägermittel aufgenommen sein, wobei das Trägermittel eine Lösung, ein Kunststoff und/oder Mikrokapseln ist. Als besonders vorteilhaft wird es angesehen, daß jeweils zwei unterschiedliche Primervarianten als Gemisch vorliegen, wobei die eine Primervariante aus der ersten und die andere Primervariante aus der zweiten Primergruppe ausgewählt ist.

Der Kit kann selbstverständlich auch weitere Primergruppen enthalten, von denen jede jeweils eine zu einem weiteren Nukleinsäuresatz komplementäre Primervariante enthält.

Das Verfahren und der Kit eignen sich insbesondere zur Markierung und Identifikation fester Substanzen, wie Zahlungsmittel, Dokumente, Datenträger und dergleichen. Gleichfalls können flüssige Stoffe, insbesondere Medikamente, Chemikalien, Lebensmittel und dergleichen, gasförmige Stoffe, insbesondere Abgase und dergleichen, markiert und identifiziert werden.

Nachfolgend wird ein Ausführungsbeispiel der Erfindung anhand der Zeichnung beschrieben. Hierin zeigen
- Fig. 1: eine schematisch dargestellte Nukleinsäuresequenz,
- Fig. 2: eine tabellarische Übersicht über die Kombinationsmöglichkeiten des zweiten Sequenzabschnitts,
- Fig. 3: eine Übersicht über die Kobinationsmöglichkeiten mit 2 x 4 Primervarianten bei der PCR,
- Fig. 4: eine schematische Darstellung des Funktionsmechanismus der Vervielfältigung der Nukleinsäuresequenzen mittels PCT,
- Fig. 5: eine Übersicht über die Kobinationsmöglichkeiten mit 2 x 4 Primervarianten bei der LCR,
- Fig. 6: eine schematische Darstellung des Funktionsmechanismus der Vervielfältigung der Nukleinsäuresequenzen mittels LCR,
- Fig. 7: die Identifizierung eines Codes und
- Fig. 8: eine schematische Darstellung des Funktionsmechanismus zur Minimierung von Basenfehlpaarungen.

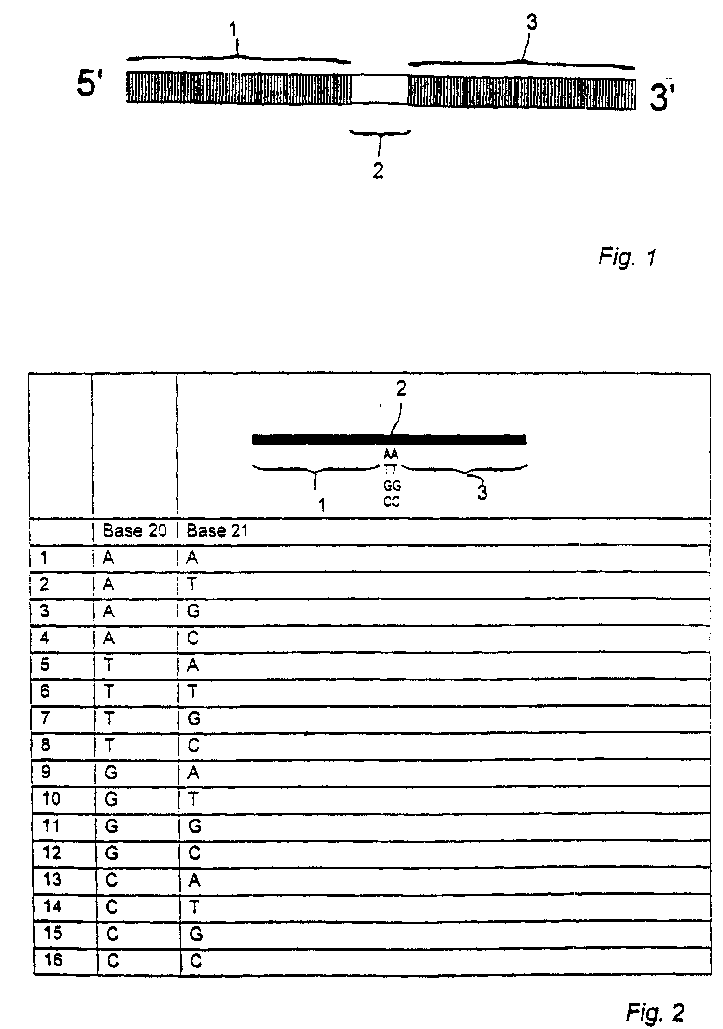

In Fig. 1 ist schematisch eine Nukleinsäuresequenz gezeigt. Sie besteht aus einem ersten das 5'-terminale Ende bildenden Sequenzabschnitt 1, einem damit verbundenen zweiten Sequenzabschnitt 2, der wiederum mit einem das 3'-terminale Ende bildenden dritten Sequenzabschnitt 3 verbunden ist.

Fig. 2 zeigt in einer tabellarischen Übersicht schematisch die sich aus einer Variation der Basen des zweiten Sequenzabschnitts ergebenden unterschiedlichen Nukleinsäurevarianten. Der erste Sequenzabschnitt 1 und der dritte Sequenzabschnitt 3 bestehen aus jeweils neunzehn Basen. Der diese beiden Sequenzabschnitte verbindende zweite Sequenzabschnitt 2 besteht aus zwei Basen. Dabei steht A für die Base Adenin, G für Guanin, C für Cytosin und T für Thymidin.

Die beiden Basen des zweiten Sequenzabschnitts besetzen die Positionen 20 und 21 der Nukleinsäuresequenz. Wie aus der Fig. 2 ersichtlich ist, können bei gleichbleibendem ersten und dritten Sequenzabschnitt 1 bzw. 3 durch unterschiedliche Besetzung der beiden Basen des zweiten Sequenzabschnitts 2 sechzehn unterschiedliche Nukleinsäurevarianten dargestellt werden. Mittels der sechzehn Nukleinsäurevarianten ist es möglich, sechzehn verschiedene Markierungen herzustellen.

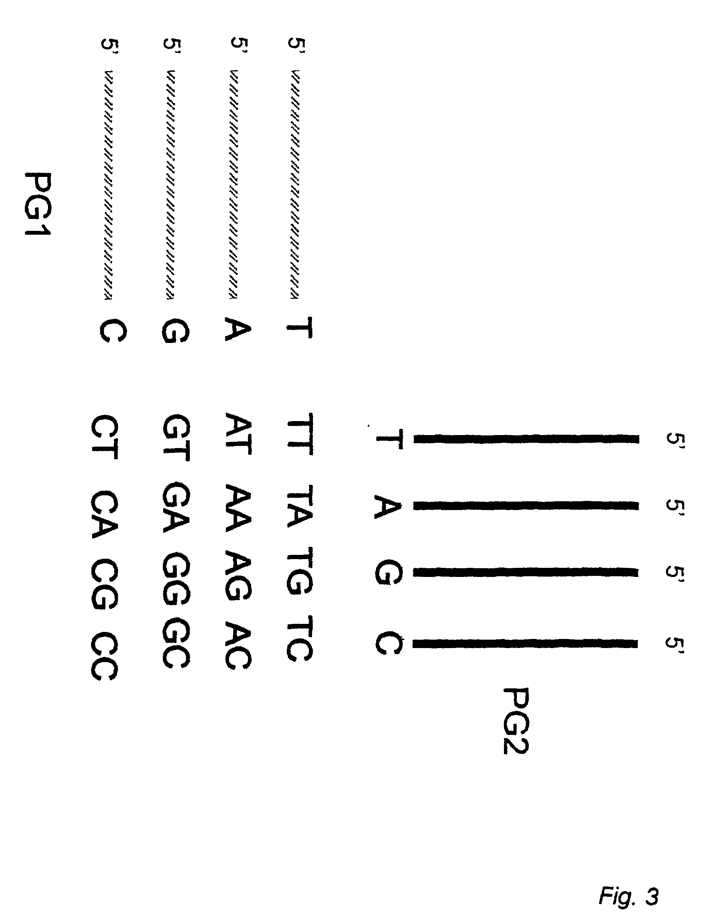

Fig. 3 beschreibt die Kombinationen, welche mit einer ersten PG1 und einer zweiten Primergruppe PG2 mit je vier Primervarianten möglich sind. Der 5'-Abschnitt der Primervarianten ist komplementär mit dem ersten bzw. dritten Abschnitt der Nukleinsäuresequenz N. Das 3'-Ende der Primervarianten trägt jeweils eine der vier Basen A, T, G, C. Durch die Kombination der Primervarianten der ersten PG1 und der zweiten Primergruppe PG2 können sich 16 verschiedene Sequenzen im zweiten Sequenzabschnitt 2 der Nukleinsäuresequenz N hybridisiert werden.

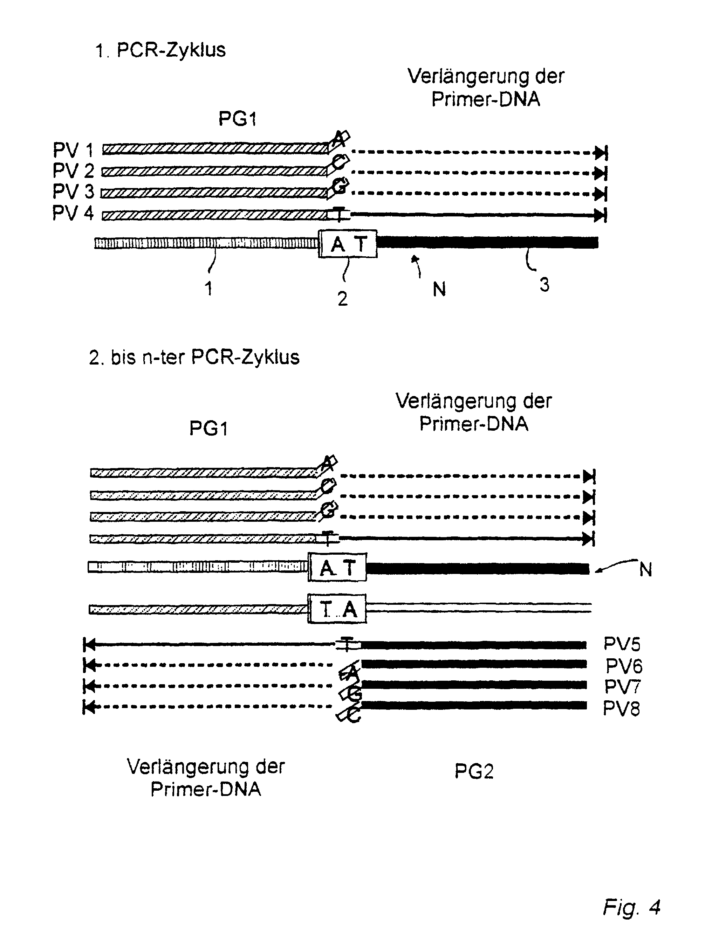

Zur Identifikation insbesondere einer sich lediglich im zweiten Sequenzabschnitt unterscheidenden Nukleinsäuresequenz wird diese zunächst in Lösung gebracht. Die Lösung wird einer PCR unterzogen. Wie aus Fig. 4 ersichtlich ist, werden dabei Primer verwendet, die nur dann mit dem ersten und dritten Sequenzabschnitt 1 bzw. 3 hybridisieren, sofern auch Übereinstimmung mit der sich daran jeweils anschließenden Base des zweiten Sequenzabschnitts 2 besteht. Zur Durchführung der PCR und damit zur Identifikation der Nukleinsäuresequenz sind demzufolge zwei Gruppen unterschiedlicher Primer erforderlich. Eine erste Primergruppe PG1 weist einen zum ersten Sequenzabschnitt 1 korrespondierenden ersten Primerabschnitt auf; eine zweite Primergruppe PG2 weist einen zum dritten Sequenzabschnitt 3 korrespondierenden dritten Primerabschnitt auf. Jede der Primergruppen PG1, PG2 umfaßt vier Primervarianten PV1 - PV8, welche sich in der jeweils endständig vorgesehenen Base unterscheiden.

Zur Identifikation wird jeweils eine Primervariante PV1 - PV4 der ersten Primergruppe PG1 mit einer Primervariante PV5 - PV8 der zweiten Primergruppe PG2 kombiniert. Es ergeben sich sechzehn mögliche Kombinationen. Die die zu identifizierende Nukleinsäuresequenz enthaltende Lösung wird einer PCR jeder der sechzehn Kombinationen unterzogen. Eine Amplifikation wird nur dort beobachtet, wo die Primerkombination eine Hybridisierung mit der zu identifizierenden Nukleinsäuresequenz erlaubt. Die Identifikation kann z.B. dadurch erfolgen, daß das Amplifikat fluoresziert. Die identifizierte Nukleinsäuresequenz kann einem vorgegebenen Zahlenwert zugeordnet werden. Dieser Zahlenwert repräsentiert den Code.

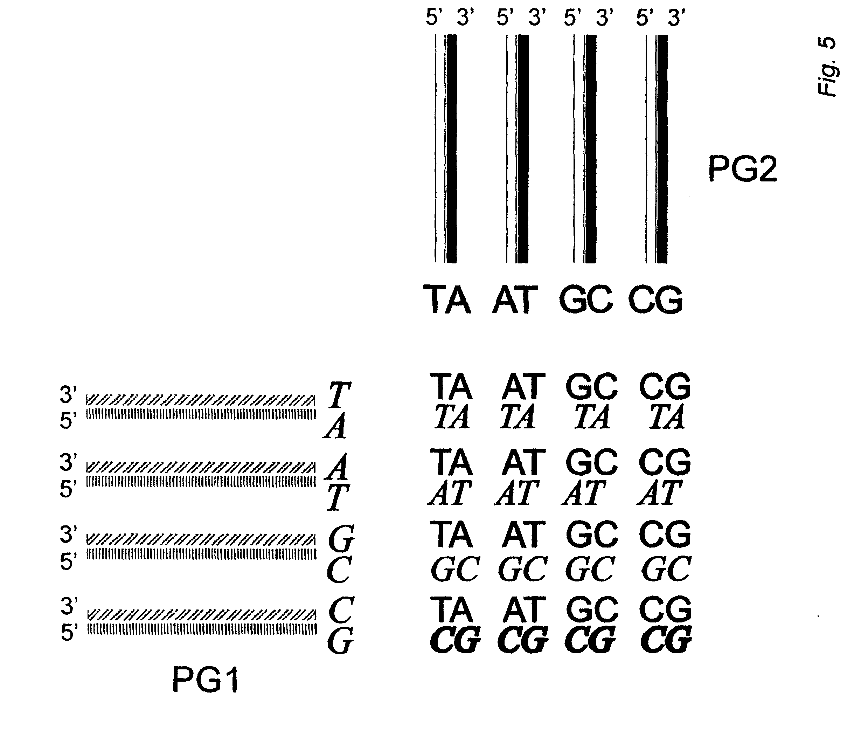

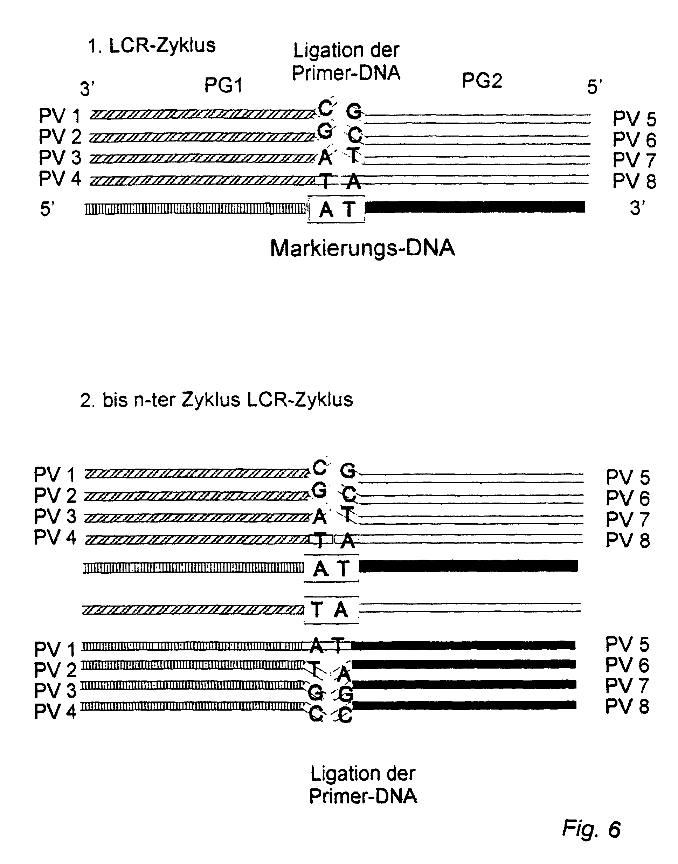

Fig. 5 und 6 zeigen die Kombinationen und den Funktionsmechanismus bei der Anwendung der LCR. Dabei werden doppelsträngige Nukleinsäuresquenzen, nämlich DNA-Sequenzen, verwendet. Zur Identifikation kommen doppelsträngige Primervarianten PV1 - PV8 zum Einsatz, die wiederum aus einer ersten PG1 und einer zweiten Primergruppe PG2 ausgewählt sind.

Im ersten Zyklus der in Fig. 6 gezeigten Identifizierungsreaktion wird der komplementäre Gegenstrang der einzelsträngigen Nukleinsäuresequenz N durch Ligation der komplementären Primervarianten PV4 und PV8 gebildet. In den weiteren Zyklen wird die Nukleinsäuresequenz amplifiziert. Da bei der Identifizierungsreaktion in jedem LCR-Ansatz nur eine der sechzehn möglichen Kombinationen der Primervarianten PV1 - PV8 vorhanden ist, kommt es nur in einer der sechzehn Reaktionsansätze zur Amplifiation.

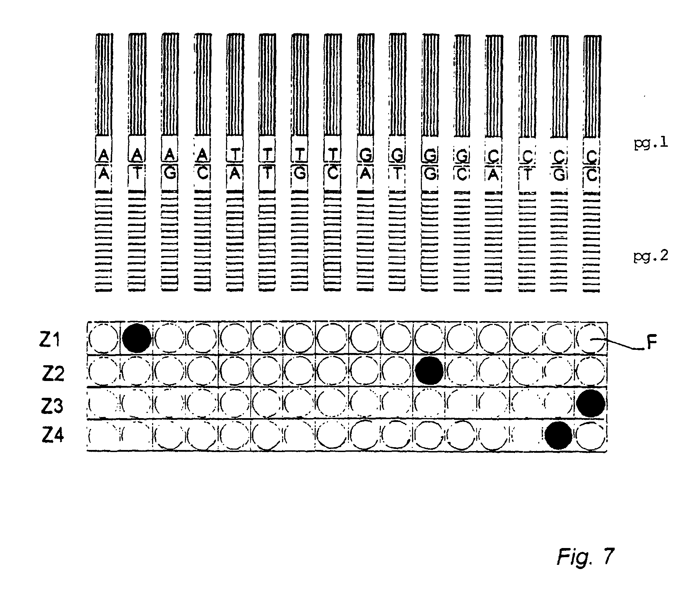

Fig. 7 zeigt beispielhaft die Identifizierung eines komplexen Codes. Der zur Markierung verwendete Stoff enthält hier vier unterschiedliche Nukleinsäuresequenzen. Jede der Nukleinsäuresequenzen ist aus einem anderen Nukleinsäuresatz ausgewählt. Jeder Nukleinsäuresatz unterscheidet sich von den anderen Nukleinsäuresätzen im ersten und dritten Sequenzabschnitt 1 bzw. 3. Die sechzehn Nukleinsäurevarianten jedes Nukleinsäuresatzes unterscheiden sich wiederum in der Besetzung der beiden Basen des zweiten Sequenzabschnitts 2.

Zur Identifikation der Markierung wird eine die vier Nukleinsäuresequenzen enthaltende Lösung auf eine Mikrotiterplatte gegeben, die vier Zeilen Z1 - Z4 mit je sechzehn Feldern F aufweist. Die 16 Proben der ersten Zeile Z1 werden zur Amplifikation mit je zwei Primervarianten versetzt, von denen eine aus einer ersten PG1 und die andere aus einer zweiten Primergruppe PG2 ausgewählt sind. Die erste Primergruppe PG1 weist einen Sequenzabschnitt auf, der korrespondierend zum ersten Sequenzabschnitt 1 einer ersten in der Probe befindlichen Nukleinsäuresequenz ist. Die zweite Primergruppe PG2 weist einen Sequenzabschnitt auf, der korrespondierend zum dritten Sequenzabschnitt 3 der ersten in der Probe befindlichen Nukleinsäuresequenz ist. Die beiden Primervarianten der ersten und zweiten Primergruppe, hybridisieren nur dann mit dem ersten und dritten Sequenzabschnitt des ersten Nukleinsäuresatzes, wenn Übereinstimmung mit der jeweiligen Base des zweiten Sequenzabschnitts 2 besteht.

Den Proben der zweiten, dritten und vierten Zeile Z2 bis Z4 werden in analoger Weise jeweils zwei Primervarianten einer dritten und vierten, fünften und sechsten sowie siebten und achten Primergruppe zugesetzt. Die vorerwähnten Primergruppen korrespondieren wiederum mit dem ersten und/oder dritten Sequenzabschnitt des zweiten, dritten und vierten Nukleinsäuresatzes.

Der Code ist so definiert, daß der jeweilige Nukleinsäuresatz die Stelle im Code bestimmt. Jeder Nukleinsäurevariante ist ein Zahlenwert zugeordnet, welcher die Wertigkeit der Stelle bestimmt. Im vorliegenden Beispiel ist der ersten Stelle im Code der Zahlwert zwei, der zweiten Stelle der Zahlenwert elf, der dritten Stelle der Zahlenwert sechzehn und der vierten Stelle der Zahlenwert fünfzehn zugeordnet.

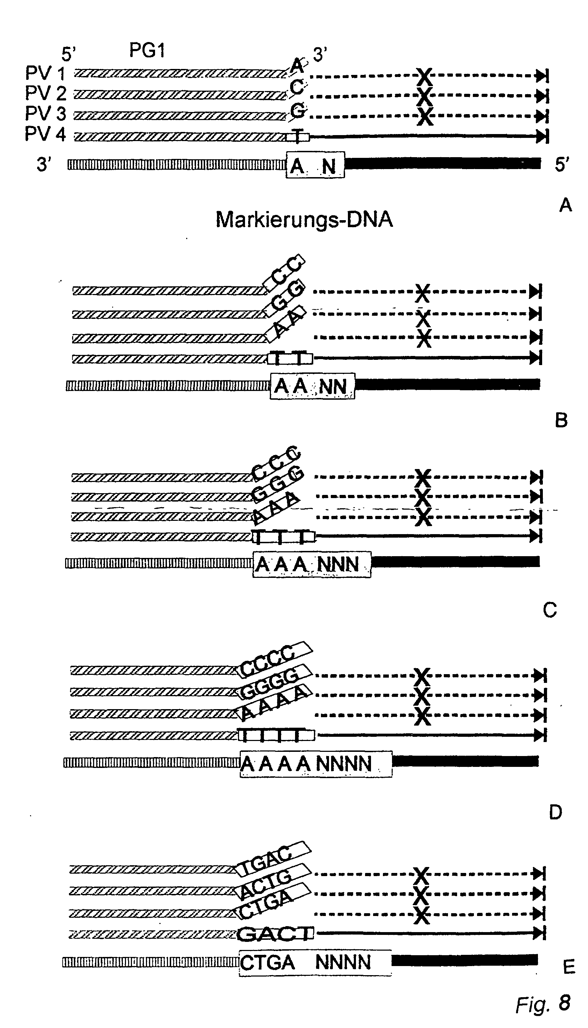

Die Fig. 8B - 8E zeigen Nukleinsäuresequenzen N, bei denen in jedem Teilbereich des zweiten Abschnitts 2 mehrere Basen vorgesehen sind. Dabei kann es sich gemäß Fig. 8B- 8D um mehrere identische Basen handeln. Wie aus Fig. 8E ersichtlich ist, sind aber auch Kombinationen möglich. Die beiden Teilbereiche des zweiten Sequenzabschnitts 2 weisen vorteilhafterweise dieselbe Länge auf.

Bei der Verwendung nur einer Base in einem Teilbereich kann es zu einem Mismatch und damit zu Primerverlängerung kommen. Um eine solche Fehlreaktion zu verhindern, kann der jeweilige Teilbereich mit einer Mehrzahl an Basen besetzt sein.

Die Anzahl der mit dem Verfahren erzeugbaren Codes nimmt mit der Anzahl der verwendeten Nukleinsäuregruppen exponentiell zu. Die Anzahl der dazu erforderlichen Nukleinsäuresequenzen steigt dagegen nur linear. Wie aus der nachfolgenden Tabelle ersichtlich ist, kann mit einer verhältnismäßig kleinen Anzahl an unterschiedlichen Nukleinsäuresequenzen eine große Anzahl an Codes realisiert werden.

| Anzahl der Nukleinsäuresätze | Anzahl der Nukleinsäurevarianten | Anzahl der erzeugbaren Codes |
|---|---|---|
| 1 | 16 = 1 x 16 | 16¹ = 16 |
| 2 | 32 = 2 x 16 | 16² = 256 |
| 3 | 48 = 3 x 16 | 16³ = 4.096 |
| 4 | 64 = 4 x 16 | 16⁴ = 65 536 |
| 5 | 80 = 5 x 16 | 16⁵ = 1.048.576 |
| 6 | 96 = 6 x 16 | 16⁶ = 16.777.216 |
| 7 | 112 = 7 x 16 | 16⁷ = 268.435.456 |
| 8 | 128 = 8 x 16 | 16⁸ = 4.294.967.296 |

Eine besonders bevorzugte Ausführungsform besteht darin, die Markierung aus 5 vorgegebenen Nukleinsäuresätzen zu bilden.

Damit können 1.048.576 unterschiedliche Markierungen bereitgestellt werden. - Zur Identifikation wird eine 96-Napf Mikrotiterplatte verwendet, bei der in jedem Napf ein anderes aus zwei Primervarianten hergestelltes Gemisch vorgelegt ist. Die Primervarianten sind jeweils mit einer fluorophoren Gruppe versehen, so daß bei Amplifikation ein Fluoreszenzsignal detektierbar ist. Aus der jeweiligen Position der Fluoreszenzsignale kann auf die Kombination der Primervarianten und damit auf die in der Markierung enthaltenen Nukleinsäuresequenz geschlossen werden. Ein solches Verfahren läßt sich ohne großen Aufwand automatisieren. Es ist kostengünstig und schnell durchführbar.

Die vorgeschlagene biologische Markierung kann auch in einen Strichcode (Bar-Code) übersetzt werden. Dabei definiert die Anzahl der vorgegebenen Nukleinsäuresätze die Anzahl der Striche der Strichcodes. Die Stelle des Strichs im Strichcode wird durch die Art des Nukleinsäuresatzes und seine Breite durch die Nukleinsäurevariante festgelegt.

## Patentansprüche

1. Verfahren zur Markierung und Identification von festen, flüssigen oder gasförmigen Substanzen, wobei die zu markierende Substanz mit mindestens einer synthetisch hergestellten Nukleinsäuresequenz (N) versehen wird, die einen ersten (1) das 5'-terminale Ende bildenden Sequenzabschnitt, einen damit verbundenen zweiten (2) aus mindestens zwei Basen (A, C, G, T) bestehenden Sequenzabschnitt und einen damit verbundenen dritten (3) das 3'-terminale Ende bildenden Sequenzabschnitt aufweist, **dadurch gekennzeichnet, daß** zur Identifikation eine erste Primergruppe (PG1) mit einem zum ersten Sequenzabschnitt (1) korrespondierenden ersten Primerabschnitt und eine zweite Primergruppe (PG2) mit einem zum dritten Sequenzabschnitt (3) korrespondierenden dritten Primerabschnitt verwendet wird, wobei jede der Primergruppen (PG1, PG2) vier, sich jeweils in mindestens einer zusätzlichen endständig vorgesehen Base (A, C, G, T) unterscheidende Primervarianten (PV1 - PV4; PV5 - PV8) umfaßt, so daß genau eine Primervariante (PV1, PV2, PV3, PV4) der ersten Primergruppe (PG1) zusammen mit genau einer Primervariante (PV5, PV6, PV7, PV8) der zweiten Primergruppe (PG2) eine Amplfikation der Nukleinsäuresequenz (N) ermöglicht.

2. Verfahren nach Anspruch 1, wobei die Nukleinsäuresequenz (N) mehr als 20 Nukleotide, vorzugsweise 40 Nukleotide, aufweist.

3. Verfahren nach einem der vorhergehenden Ansprüche, wobei eine Mehrzahl an Nukleinsäuresequenzen (N) verwendet wird, die im zweiten Sequenzabschnitt (2) sich unterscheiden.

4. Verfahren nach einem der vorhergehenden Ansprüche, wobei der zweite Sequenzabschnitt (2) aus zwei Teilbereichen besteht, von denen mindestens einer aus einer Mehrzahl von Basen (A, C, G, T) besteht.

5. Verfahren nach einem der vorhergehenden Ansprüche, wobei die Nukleinsäuresequenzen im ersten (1) und dritten Sequenzabschnitt (3) sich unterscheiden.

6. Verfahren nach einem der vorhergehenden Ansprüche, wobei der erste (1) und der dritte Sequenzabschnitt (3) die gleiche Anzahl an Nukleotiden aufweisen.

7. Verfahren nach einem der vorhergehenden Ansprüche, wobei der erste (1) und der dritte Sequenzabschnitt (3) unter vergleichbaren Stringenzbedingungen aufschmelzbar sind.

8. Verfahren nach einem der vorhergehenden Ansprüche, wobei die Primervarianten (PV1 - PV8) unter vergleichbaren Stringenzbedingungen aufschmelzbar sind.

9. Verfahren nach einem der vorhergehenden Ansprüche, wobei als Nukleinsäuresequenz (N) eine Nukleinsäurederivatsequenz, insbesondere proteinartige Nukleinsäure (PNA) oder Phosphothionatnukleinsäuren (PTO) oder Hybride davon, verwendet wird.

10. Verfahren nach einem der vorhergehenden Ansprüche, wobei jeder zu markierende Substanz mit mindestens einer unterscheidbaren Nukleinsäuresequenz (N) versehen wird.

11. Verfahren nach einem der vorhergehenden Ansprüche, wobei die auf feste Gegenstände aufgetragene Nukleinsäuresequenz (N) durch eine Schutzschicht abgedeckt wird.

12. Verfahren nach einem der vorhergehenden Ansprüche, wobei die Nukleinsäuresequenz (N) Bestandteil einer auf feste Gegenstände aufgetragenen Schicht ist.

13. Verfahren nach einem der vorhergehenden Ansprüche, wobei die zu markierende Substanz mit der Nukleinsäuresequenz (N) imprägniert wird.

14. Verfahren nach einem der vorhergehenden Ansprüche, wobei die Nukleinsäuresequenz (N) dem zu markierenden Stoff beigemischt wird.

15. Verfahren nach einem der vorhergehenden Ansprüche, wobei zur Identifikation der Markierung die Nukleinsäuresequenz (N) von der Substanz bzw. dem Gegenstand extrahiert oder entfernt wird.

16. Verfahren nach einem der vorhergehenden Ansprüche, wobei eine die extrahierte oder entfernte Nukleinsäuresequenz (N) enthaltende Lösung hergestellt wird.

17. Verfahren nach einem der vorhergehenden Ansprüche, wobei die in der Lösung enthaltene Nukleinsäuresequenz (N) unter Verwendung der Primervarianten (PV1 - PV8) mittels Polymerase-Ketten-Reaktion (PCR) vervielfältigt werden.

18. Verfahren nach einem der vorhergehenden Ansprüche, wobei die in der Lösung enthaltene Nukleinsäuresequenz (N) unter Verwendung der Primervarianten (PV1 - PV8) mittels Ligase-Ketten-Reaktion (LCR) vervielfältigt wird.

19. Verfahren nach einem der vorhergehenden Ansprüche, wobei die vervielfältigte Nukleinsäuresequenz (N) mittels Fluoreszenz nachgewiesen wird.

20. Kit zur Identifikation einer Markierung nach Anspruch 1, **dadurch gekennzeichnet, daß** der Kit eine erste Primergruppe (PG1) mit einem zum ersten Sequenzabschnitt (1) korrespondierenden ersten Primerabschnitt und eine zweite Primergruppe (PG2) mit einem zum dritten Sequenzabschnitt (3) korrespondierenden dritten Primerabschnitt aufweist, wobei jede der Primergruppen (PG1, PG2) vier sich jeweils in mindestens einer zusätzlichen endständig vorgesehen Base (A, C, G, T) unterscheidende Primervarianten (PV1 - PV4; PV5 - PV8) umfaßt, so daß genau eine Primervariante (PV1, PV2, PV3, PV4) der ersten Primergruppe (PG1) zusammen mit genau einer Primervariante (PV5, PV6, PV7, PV8) der zweiten Primergruppe (PG2) zur Nukleinsäuresequenz (N) komplementär ist.

21. Kit nach Anspruch 20, wobei die Primervarianten (PV1 - PV4; PVS - PV8) dieselbe oder eine ähnliche Anzahl an Nukleotiden aufweisen.

22. Kit nach einem der Ansprüche 20 oder 21, wobei die Primervarianten (PV1 - PV4; PV5 - PV8) mehr als 10 Nukleotide, vorzugsweise 20 Nukleotide, aufweisen.

23. Kit nach einem der Ansprüche 20 bis 22, wobei die Nukleinsäuresequenzen im zweiten Sequenzabschnitt (2) sich unterscheiden.

24. Kit nach einem der Ansprüche 20 bis 23, wobei die jeder der Primervarianten (PV1 - PV8) separat von einem Trägermittel aufgenommen ist.

25. Kit nach Anspruch 24, wobei das Trägermittel eine Lösung, eine Mikrotiterplatte, ein Kunststoff und/oder Mikrokapseln ist.

26. Kit nach einem der vorhergehenden Ansprüche 20 bis 25, wobei jeweils zwei unterschiedliche Primervarianten (PV1 - PV8) als Gemisch an oder in einem Trägermittel aufgenommen sind.

27. Kit nach einem der vorhergehenden Ansprüche 20 bis 26, wobei die Primervarianten (PV1 - PV8) mit einer fluorophoren Gruppe versehen sind, so daß bei Amplifikation ein Fluoreszenzsignal detektierbar ist.

## Claims

1. Process for marking and identification solid, liquid or gaseous substances, where the substance to be marked is provided with at least one synthetically prepared nucleic acid sequence (N) which has a first (1) sequence section forming the 5'-terminal end, a second (2) sequence section consisting of at least two bases (A, C, G, T) connected therewith and a third (3) sequence section forming the 3'-terminal end connected therewith, **characterized in that**, for identification, a first primer group (PG1) having a first primer section corresponding to the first sequence section (1) and a second primer group (PG2) having a third primer section corresponding to the third sequence section (3) is used, where each of the primer groups (PG1, PG2) comprises four primer variants (PV1 - PV4; PV5 - PV8), in each case differing in at least one additional terminally provided base (A, C, G, T), such that just one primer variant (PV1, PV2, PV3, PV4) of the first primer group (PG1) together with just one primer variant (PV5, PV6, PV7, PV8) of the second primer group (PG2) makes possible amplification of the nucleic acid sequence (N).

2. Process according to claim 1, where the nucleic acid sequence (N) has more than 20 nucleotides, preferably 40 nucleotides.

3. Process according to one of the preceding claims, where a majority of nucleic acid sequences (N) is used which differ in the second sequence section (2).

4. Process according to one of the preceding claims, where the second sequence section (2) consists of two part regions, of which at least one consists of a majority of bases (A, C, G, T).

5. Process according to one of the preceding claims, where the nucleic acid sequences in the first (1) and third sequence section (3) differ.

6. Process according to one of the preceding claims, where the first (1) and the third sequence section (3) have the same number of nucleotides.

7. Process according to one of the preceding claims, where the first (1) and the third sequence section (3) are fusible under comparable stringency conditions.

8. Process according to one of the preceding claims, where the primer variants (PV1 - Pv8) are fusible under comparable stringency conditions.

9. Process according to one of the preceding claims, where the nucleic acid sequence (N) used is a nucleic acid derivative sequence, in particular protein-like nucleic acid (PNA) or phosphothionate nucleic acids (PTO) or hybrids thereof.

10. Process according to one of the preceding claims, where each substance to be marked is provided with at least one distinguishable nucleic acid sequence (N).

11. Process according to one of the preceding claims, where the nucleic acid sequence (N) applied to solid articles is covered by a protective layer.

12. Process according to one of the preceding claims, where the nucleic acid sequence (N) is constituent of a layer applied to solid articles.

13. Process according to one of the preceding claims, where the substance to be marked is impregnated with the nucleic acid sequence (N).

14. Process according to one of the preceding claims, where the nucleic acid sequence (N) is admixed to the substance to be marked.

15. Process according to one of the preceding claims, where, for identification of the marking, the nucleic acid sequence (N) is extracted or removed from the substance or the article.

16. Process according to one of the preceding claims, where a solution containing the extracted or removed nucleic acid sequence (N) is prepared.

17. Process according to one of the preceding claims, where the nucleic acid sequence (N) contained in the solution is duplicated by means of polymerase chain reaction (PCR) using the primer variants (PV1 - PV8).

18. Process according to one of the preceding claims, where the nucleic acid sequence (N) contained in the solution is duplicated by means of ligase chain reaction (LCR) using the primer variants (PV1 - PV8).

19. Process according to one of the preceding claims, where the duplicated nucleic acid sequence (N) is detected by means of fluorescence.

20. Kit for the identification of a marking according to claim 1, **characterized in that** the kit has a first primer group (PG1) having a first primer section corresponding to the first sequence section (1) and a second primer group (PG2) having a third primer section corresponding to the third sequence section (3), where each of the primer groups (PG1, PG2) comprises four primer variants (PV1 - PV4; PV5 - PV8), in each case differing in at least one additional terminally provided base (A, C, G, T), such that just one primer variant (PV1, PV2, V3, PV4) of the first primer group (PG1) together with just one primer variant (PV5, PV6, PV7, PV8) of the second primer group (PG2) is complementary to the nucleic acid sequence (N).

21. Kit according to claim 20, where the primer variants (PV1 - PV4; PV5 - PV8) have the same or a similar number of nucleotides.

22. Kit according to either of claims 20 or 21, where the primer variants (PV1 - PV4; PV5 - PV8) have more than 10 nucleotides, preferably 20 nucleotides.

23. Kit according to one of claims 20 to 22, where the nucleic acid sequences differ in the second sequence section (2).

24. Kit according to one of claims 20 to 23, where each of the primer variants (PV1 - PV8) is taken up separately by a carrier.

25. Kit according to claim 24, where the carrier is a solution, a microtiter plate, a plastic and/or microcapsules.

26. Kit according to one of the preceding claims 20 to 25, where two different primer variants (PV1 - PV8) are in each case taken up as a mixture on or in a carrier.

27. Kit according to one of the preceding claims 20 to 26, where the primer variants (PV1 - PV8) are provided with a fluorophoric group, such that a fluorescence signal is detectable on amplification.

## Revendications

1. Procédé pour le marquage et la identification de substances solides, liquides ou gazeuses, la substance à marquer étant pourvue d'au moins une séquence d'acide nucléique (N) produite par synthèse, qui comporte un premier segment de séquence (1) constituant l'extrémité 5'-terminale, un deuxième segment de séquence (2) relié à celui-ci, constitué d'au moins deux bases (A, C, G, T), et un troisième segment de séquence (3) relié à ce dernier, constituant l'extrémité 3'-terminale, **caractérisé en ce que**, pour l'identification, on utilise un premier groupe d'amorces (PG1) comportant un premier segment d'amorce correspondant au premier segment de séquence (1) et un second groupe d'amorces (PG2) comportant un troisième segment d'amorce correspondant au troisième segment de séquence (3), chacun des groupes d'amorces (PG1, PG2) comprenant quatre variantes d'amorce (PV1-PV4 ; PV5-PV8) différant chacune par au moins une base (A, C, G, T) supplémentaire prévue en bout de chaîne, de sorte qu'exactement une variante d'amorce (PV1, PV2, PV3, PV4) du premier groupe d'amorces (PG1), conjointement avec exactement une variante d'amorce (PV5, PV6, PV7, PV8) du second groupe d'amorces (PG2) permet une amplification de la séquence d'acide nucléique (N).

2. Procédé selon la revendication 1, dans lequel la séquence d'acide nucléique (N) comprend plus de 20 nucléotides, de préférence 40 nucléotides.

3. Procédé selon l'une des revendications précédentes, dans lequel on utilise une multiplicité de séquences d'acide nucléique (N) qui diffèrent entre elles par le deuxième segment de séquence (2).

4. Procédé selon l'une des revendications précédentes, dans lequel le deuxième segment de séquence (2) consiste en deux régions partielles, dont au moins une consiste en une multiplicité de bases (A, C, G, T).

5. Procédé selon l'une des revendications précédentes, dans lequel les séquences d'acide nucléique diffèrent entre elles dans le premier segment de séquence (1) et le troisième segment de séquence (3).

6. Procédé selon l'une des revendications précédentes, dans lequel le premier segment de séquence (1) et le troisième segment de séquence (3) comportent le même nombre de nucléotides.

7. Procédé selon l'une des revendications précédentes, dans lequel le premier segment de séquence (1) et le troisième segment de séquence (3) peuvent être fondus dans des conditions comparables de stringence.

8. Procédé selon l'une des revendications précédentes, dans lequel les variantes d'amorces (PV1-PV8) peuvent être fondues dans des conditions comparables de stringence.

9. Procédé selon Tune des revendications précédentes, dans lequel on utilise comme séquence d'acide nucléique (N) une séquence de dérivé d'acide nucléique, en particulier de l'acide nucléique de type protéine (PNA) ou des acides nucléiques dérivés en phosphothionate (PTO), ou des hybrides de ceux-ci.

10. Procédé selon l'une des revendications précédentes, dans lequel chaque substance à marquer est pourvue d'au moins une séquence nucléique (N) pouvant être distinguée.

11. Procédé selon l'une des revendications précédentes, dans lequel la séquence d'acide nucléique (N) appliquée sur des objets solides est recouverte d'une couche protectrice.

12. Procédé selon Tune des revendications précédentes, dans lequel la séquence d'acide nucléique (N) fait partie d'une couche appliquée sur des objets solides.

13. Procédé selon Tune des revendications précédentes, dans lequel la substance à marquer est imprégnée de la séquence d'acide nucléique (N).

14. Procédé selon l'une des revendications précédentes, dans lequel la séquence d'acide nucléique (N) est ajoutée à la substance à marquer.

15. Procédé selon l'une des revendications précédentes, dans lequel, pour l'identification du marquage, la séquence d'acide nucléique (N) est éliminée ou extraite de la substance ou de l'objet.

16. Procédé selon l'une des revendications précédentes, dans lequel on prépare une solution contenant la séquence d'acide nucléique (N) extraite ou éliminée.

17. Procédé selon l'une des revendications précédentes, dans lequel la séquence d'acide nucléique (N) contenue dans la solution est amplifiée par une réaction d'amplification en chaîne par polymérase (PCR), avec utilisation des variantes d'amorces (PV1-PV8).

18. Procédé selon l'une des revendications précédentes, dans lequel la séquence d'acide nucléique (N) contenue dans la solution est amplifiée par une réaction d'amplification en chaîne par ligase (LCR), avec utilisation des variantes d'amorces (PV1-PV8).

19. Procédé selon l'une des revendications précédentes, dans lequel la séquence d'acide nucléique (N) amplifiée est détectée par fluorescence.

20. Nécessaire pour l'identification d'un marquage selon la revendication 1, **caractérisé en ce que** le nécessaire comporte un premier groupe d'amorces (PG1) comportant un premier segment d'amorce correspondant au premier segment de séquence (1) et un second groupe d'amorces (PG2) comportant un troisième segment d'amorce correspondant au troisième segment de séquence (3), chacun des groupes d'amorces (PG1, PG2) comprenant quatre variantes d'amorce (PV1-PV4 ; PV5-PV8) différant chacune par au moins une base (A, C, G, T) supplémentaire prévue en bout de chaîne, de sorte qu'exactement une variante d'amorce (PV1, PV2, PV3, PV4) du premier groupe d'amorces (PG1), conjointement avec exactement une variante d'amorce (PV5, PV6, PV7, PV8) du second groupe d'amorces (PG2) est complémentaire de la séquence d'acide nucléique (N).

21. Nécessaire selon la revendication 20, dans lequel les variantes d'amorces (PV1-PV4 ; PV5-PV8) comportent le même nombre ou un nombre similaire de nucléotides.

22. Nécessaire selon la revendication 20 ou 21, dans lequel les variantes d'amorces (PV1-PV4 ; PV5-PV8) comportent plus de 10 nucléotides, de préférence 20 nucléotides.

23. Nécessaire selon l'une des revendications 20 à 22, dans lequel les séquences d'acide nucléique diffèrent dans le second segment de séquence (2).

24. Nécessaire selon l'une des revendications 20 à 23, dans lequel chacune des variantes d'amorces (PV1-PV8) est capturée séparément par un support.

25. Nécessaire selon la revendication 24, dans lequel le support est une solution, une plaque de microtitrage, une matière plastique et/ou des microcapsules.

26. Nécessaire selon l'une des revendications précédentes 20 à 25, dans lequel deux variantes d'amorces différentes (PV1-PV8) sont chaque fois capturées en tant que mélange sur ou dans un support.

27. Nécessaire selon l'une des revendications précédentes 20 à 26, dans lequel les variantes d'amorces (PV1-PV8) sont pourvues d'un groupe fluorophore, de sorte qu'un signal de fluorescence est détectable lors de l'amplification.
